# EUROPEAN PATENT SPECIFICATION

(11) **EP 0 643 970 B1**
(45) Date of publication and mention of the grant of the patent: **16.08.2001**
(21) Application number: 94118300.6
(22) Date of filing: 16.04.1985
(51) Int. Cl.: A61K 31/70

(54) **The use of 1-beta-D-ribofuranosyl-1,2,4-triazole-3-carboxamide (Ribavirin) for the preparation of a medicament for the treatment of arboviruses**
Verwendung von 1-Beta-D-ribofuranosyl-1,2,4-triazol-3-carboxamid (Ribavirin) zur Herstellung eines Medikaments für die Behandlung von Arboviren
Utilisation du 1-Beta-D-ribofuranosyl-1,2,4-triazole-3-carboxamide (Ribavirin) pour l'obtention d'un médicament destiné au traitement des arboviruses

(30) Priority: 29.10.1984 US 666252
(43) Date of publication of application: 22.03.1995
(62) Divisional of application: 90105678.8
(73) Proprietor: VIRATEK, INC., Costa Mesa California 92626 (US)
(72) Inventor: Smith, Robert Angus, Santa Monica, California 90402 (US); Fernandes, Humberto, Irvine, California 92714 (US)
(74) Representative: Viering, Jentschura & Partner

(56) References cited:
- WO-A-83/03383
- ABSTR.ANNU.MEET.AM.SOC.MICROBIOL., 1979, page 275 XP002026770 D.E.JONES ET AL.: "Rift Valley Fever Infected C57Bl6 Mice: Evaluation of the Antiviral Activity of Ribavirin"
- ABSTR.ANNU.MEET.AM.SOC.MICROBIOL., 1982, page 15, abstract A84 XP002026771 B.J.LUSCRI: "Synergy Between Human Beta Interferon (IFN) and Ribavirin (RVN)."
- ABSTR.ANNU.MEET.AM.SOC.MICROBIOL., 1980, page 257, abstract T 134 XP002026772 P.B.JAHRLING ET AL.: "Pathogenesis and Treatment of Lassa (LAS) and Pichinde (PIC) Virus Infections in Guinea Pigs"
- DRUGS, vol. 22, no. 2, August 1981, pages 111-128, XP002026773 T.-W. CHANG ET AL.: "Ribavirin and Inosiplex: A Review of their Present Status in Viral Diseases"
- J.ANTIMICROB.CHEMOTHER., vol. 14, no. Suppl. A, August 1984, pages 27-41, XP002026774 P.G.CANONICO ET AL.: "In-vivo activity of antivirals against exotic RNA viral infections"
- ANTIMICROB.AGENTS CHEMOTHER., vol. 26, no. 4, October 1984, pages 476-480, XP002026775 J.W.HUGGINS ET AL.: "Synergistic Antiviral Effects of Ribavirin and the C-Nucleoside Analogs Tiazofurin and Selenazofurin Against Togaviruses, Bunyaviruses, and Arenaviruses"
- ANTIVIRAL RES., vol. 2, no. 1-2, May 1982, pages 69-79, XP002026776 W.C.KOFF ET AL.: "ANTIVIRAL EFFECTS OF RIBAVIRIN AND 6-MERCAPTO-9-TETRAHYDRO-2-FURYLPURINE AGAINST DENGUE VIRUSES IN VITRO"
- ANTIMICROB.AGENTS CHEMOTHER., vol. 24, no. 1, July 1983, pages 134-136, XP002026777 W.C.KOFF ET AL.: "Treatment of Intracranial Dengue Virus Infections in Mice with a Lipophilic Derivative of Ribavirin"
- DIALOG INFORMATION SERVICES, FILE 399: CA SEARCH (R), ACCESSION NUMBER 93161332, XP002026778 & RIBAVIRIN: BROAD SPECTRUM ANTIVIRAL AGENT, R.A.SMITH ET AL. (ED.), 1980, pages 169-183, E.L.STEPHEN ET AL.: "Ribavirin treatment of toga-, arena- and bunyavirus infections in subhuman primates and other laboratory animal species"
- ANTIVIRAL RES., vol. 2, no. 6, December 1982, pages 331-337, XP002026779 P.G.CANONICO ET AL.: "ANTIVIRAL EFFICACY OF PYRAZOFURIN AGAINST SELECTED RNA VIRUSES"
- VIROLOGY, vol. 102, no. 2, 1980, pages 473-476, XP002026780 F.MALINOSKI ET AL.: "Inhibition of Sindbis Virus Replication in Aedes albopictus Cells by Virazole (Ribavirin) and its Reversal by Actinomycin: A Correction"
- ANTIMICROB.AGENTS CHEMOTHER., vol. 24, no. 3, September 1983, pages 353-361, XP002026781 J.J.KIRSI ET AL.: "Broad-Spectrum Antiviral Activity of 2-beta-D-Ribofuranosylselenazole-4-Carboxa mide, a New Antiviral Agent"
- J.MED.CHEM., vol. 25, no. 11, 1982, pages 1334-1338, XP002026784 R.J.GOEBEL ET AL.: "Synthesis and Antiviral Activity of Certain Carbamoylpyrrolopyrimidine and Pyrazolopyrimidine Nucleosides"
- SOUTHEAST ASIAN J. TROP. MED. PUB. HLTH., vol. 12, no. 3, 1981, page 436 XP002026785 M.A.USSERY: "EFFECT OF THE ANTIVIRAL DRUG RIBAVIRIN ON DENGUE VIRUS REPLICATION IN LLC-MK2 CELLS"
- DIALOG INFORMATION SERVICES, FILE 155 MEDLINE (R), ACCESSION NUMBER 04334527 XP002026787 & VOPR. VIRUSOL., vol. 28, no. 5, September 1983 - October 1983, USSR, pages 627-629, L.K.BEREZINA ET AL: "Effect of ribavirin on bunyavirus reproduction in cell culture and in an experiment on white mice"
- LANCET, vol. 1, no. 8110, 3 February 1979, pages 268-269, XP000673226 E.L.STEPHEN ET AL.: "EXPERIMENTALLY LASSA FEVER VIRUS INFECTION SUCCESSFULLY TREATED WITH RIBAVIRIN"
- LANCET, vol. 2, no. 8405, 29 September 1984, pages 728-729, XP000673201 [EDITORIAL]: "Management of Lassa Fever"
- LANCET, vol. 2, no. 8402, 8 September 1984, pages 562-564, XP000673200 K.G.NICHOLSON: "Antiviral Agents in Clinical Practice, PROPERTIES OF ANTIVIRAL AGENTS, Second of Two Parts"
- ABSTR.ANNU.MEET.AM.SOC.MICROBIOL., vol. 78, 1978, page 228 XP000671647 R.W.KUEHNE ET AL.: "Treatment of Tacaribe Virus Infection of Mice Using Various Antiviral Compounds"
- ANTIMICROB. AGENTS CHEMOTHER., vol. 24, no. 5, November 1983, pages 696-701, XP002030796 R.K.AUSTIN ET AL.: "Sensitive Radioimmunoassay for the Broad-Spectrum Antiviral Agent Ribavirin"
- TOXICOL.APPL.PHARMACOL., vol. 64, no. 1, 1982, pages 155-159, XP002030797 G.L.WANNARKA ET AL.: "Preclinical Evaluation in Monkeys of a Ribavirin Regimen Proposed for Use in Lassa Fever Patients"
- J.INFECT.DIS., vol. 141, no. 5, May 1980, pages 580-589, XP002030798 P.B.JAHRLING ET AL.: "Lassa Virus Infection of Rhesus Monkeys: Pathogenesis and Treatment with Ribavirin"
- J.INFECT.DIS., vol. 149, no. 3, March 1984, pages 420-427, XP002030799 P.N.JAHRLING ET AL.: "Enhanced Treatment of Lassa Fever by Immune Plasma Combined with Ribavirin in Cynomolgus Monkeys"
- DIALOG INFORMATION SERVICES, FILE 399: CA SEARCH(R), ACCESSION NUMBER 101083620, XP002030800 & PROBL.PERSPEKT. IZUCH. NEKLEOZIDOV, BITSIKLOGEPTANA, ADAMANTANA DRUGIKH PROTIVOVIRUSN. SOEDIN. EKSP. KLIN., 1982, pages 27-31, A.S.PETKEVICH ET AL.: "Study of the effect of ribavirin on Lassa Virus reproduction in cell culture"
- DIALOG INFORMATION SERVICES, FILE 5: BIOSIS PREVIEWS(R), ACCESSION NUMBER 3737825, XP002030801 & VOPR. VIRUSOL., vol. 0, no. 2, 1981, pages 244-245, A.S.PETKEVICH ET AL.: "EFFECT OF RIBOVIRIN VIRAZOLE ON THE REPRODUCTION OF SOME ARENAVIRUSES IN A CELL CULTURE"
- J.ANTIMICROB.CHEMOTHER., vol. 15, no. 2, February 1985, pages 129-131, XP002030802 P.G.CANONICO ET AL.: "Chemotherapy for 'exotic' RNA viruses"
- ISHII T. ET AL.: "Effective Ribavirin Concentration in Hamster Brains for Antiviral Chemotherapy for Subacute Sclerosing Panencephalitis", ANTIMICROB AGENTS CHEMOTHER, , 01. January 1996, vol. 40, no. 1, pages 241 to 243

## Description

The invention involves the use of Ribavirin (1-β-D-ribofuranosyl-1,2,4-triazole-3-carboxamide) for the manufacture of a medicament for treating viral diseases in humans, caused by Arboviruses excluding Bunyaviruses, wherein Ribavirin is contained in said medicament in a total dose from 8.5 to 50 milligrammes per kilogramme of body weight per day.

### Background

The ongoing search for pharmacologic agents which are effective against various disease states caused by the over three hundred different immunologic types of virus is an important one. Viral disease accounts for some 60% of all diseases in humans. Among those viral disease states for which cures have been sought are:
(a) bronchiolitis, bronchitis, bronchopneumonia, tracheobronchitis, croup and febrile respiratory disease associated with Respiratory Syncytial Virus (RSV);
(b) Lassa Fever associated with hemorrhage, fever, pharyngitis, enteritis, myocarditis, pneumonitis, and, in fatal cases, circulatory collapse;
(c) Korean Hemorrhagic Fever associated with Arenaviruses and its clinical manifestations including severe toxemia, acute febrile illness, widespread capillary damage, hemorrhagic phenomena, severe central nervous system symptomatology and renal insufficiency (nephrosonephritis);
(d) Neoplasms, tumors, infections, and conditions such as Acquired Immune Deficiency Syndrome (AIDS) which is characterized by the development of Kaposi's Sarcoma and various infections; leukemias and lymphomas, associated with Retroviruses (RNA Tumor Viruses) and Adenoviruses;
(e) Viral infections in immunocompromised patients.

As a result of the significant morbidity and mortality of these viral diseases and the fact that efforts to develop an effective vaccine have thus far been unsuccessful, it is apparent that a chemotherapeutic agent effective against these disease states is needed. The search for effective antiviral pharmacotherapeutic agents is further complicated by the significant development of drug resistance by the various viruses to antiviral agents other than 1-β-D-ribofuranosyl-1, 2, 4-triazole-3-carboxamide.

### Brief Summary of the Invention

The present invention involves the use of the compound 1-β-D-ribofuranosyl-1, 2, 4-triazole-3-carboxamide in the treatment of disease states caused by Arboviruses excluding Bunyaviruses various viruses in humans. 1-β-D-ribofuranosyl-1, 2, 4-triazole-3-carboxamide has been shown to exhibit broad spectrum antiviral activity both in vitro and in vivo. The compound may be administered either by injection, orally, topically, ophthalmically or via sprays or aerosol into the respiratory tract. Furthermore, the compound may be administered alone or in combination with other pharmacologic agents depending upon the disease state being treated.

### Detailed Description of the Invention

United States Patent No. 3,798,209, which reissued November 14, 1978 as Re 29,835, disclosed the compound 1-β-Dribofuranosyl-1, 2, 4-triazole-3-carboxamide as an antiviral agent exhibiting broad spectrum antiviral activity both in vitro and in vivo. Medical use of the compound in specific viral disease states was disclosed in United States Patent No. 4,211,771.

Below is a detailed description of the use of the compound 1-β-D-ribofuranosyl-1, 2, 4-triazole-3-carboxamide alone or in combination with other pharmacologic agents in the treatment of various viral diseases caused by Arboviruses except Bunyaviruses. For the purposes of further illustrating the invention, 1-β-D-ribofuranosyl-1,2,4triazole-3-carboxamide will be referred to interchangeably as (a) the compound, (b) Ribavirin (non-proprietary name adopted by the United States Adopted Names Council), or (c) under its chemical name above.

For example, Respiratory Syncytial Virus (RSV) which does not belong to the group of Arboviruses was first isolated in 1956 and was subsequently found to be the major lower respiratory tract pathogen of infancy and early childhood throughout the world. (Chanock, R.M., et al., Acute Respiratory Disease in Infancy and Childhood: Present Understanding and Prospects for Prevention. Pediatrics, Vol. 36, pages 21-39 (1965))

Of particular concern, but not limited only to, are infections in immunocompromised patients. For example, infants with Severe Combined Immunodeficiency Syndrome (SCID) are subject to overwhelming or prolonged infections with a number of viruses including RSV, influenza, parainfluenza and other RNA viruses. (Jarvis, W.R., et al., Significance of Viral Infections in Severe Combined Immunodeficiency Disease-, Pediatr Infect Dis, Volume 2, pages 187-192 (1983))

As a result of the potential severity of symptomatology, occurrence and recurrence of these viral infections, the importance of finding a specific chemotherapeutic agent for the treatment of patients so infected is apparent. This concern is even further highlighted by the fact that efforts to develop an effective vaccine have thus far been unsuccessful. (Taber, L.H., et al., Ribavarin Aerosol Treatment of Bronchiolitis Associated with Respiratory Syncytial Virus Infection in Infants. Pediatrics, Volume 72, No. 5, pages 613-618 (November, 1983)).

The importance of finding an effective antiviral agent which is not subject to the development of drug resistance cannot be overemphasized in immunocompromised patients. Patients with compromised immune systems or with congenital heart and/or lung disease may require frequent and prolonged drug therapies thereby making conditions ripe for the development of drug resistance in susceptible agents. The development of resistant viral strains in the treatment of various viral diseases with, for example, Acyclovir, is a continuing problem. (See, Whittington W.L., et al., Acyclovir Therapy For Genital Herpes: Enthusiasm and Caution in Equal Doses, JAMA, Vol. 251 No. 16, April 27, 1984.) Drug resistance has not been found to occur with Ribavirin.

Arenaviruses produce highly lethal hemorrhagic fevers, although milder and subclinical infections quite commonly occur also. While showing fewer hemorrhagic manifestations than, for example, Argentinian-Bolivian Hemorrhagic Fever, Lassa Fever causes severe pharyngitis, enteritis, myocarditis, and pneumonitis, and in fatal cases, circulatory collapse. (Fenner, F.J., et al., Medical Virology Second Edition, pages 416-417 (1976).) The etiologic agent in Lassa Fever is an Arenavirus endemic to regions of West Africa. The overall mortality rate of hospitalized cases reaches 15 to 20%.

For example, Korean Hemorrhagic Fever also known as Hemorrhagic Fever with Renal Syndrome, Far Eastern Hemorrhagic Fever, endemic or epidemic nephrosonephritis, Manchurian Epidemic Hemorrhagic Fever, Songo Fever and Churilov's Disease, suggests an Arenavirus or Bunyavirus etiology. The hemorrhagic fever associated with KHF is more severe than that seen in Lassa Fever. Additionally, KHF is associated with a renal syndrome known as hemorrhagic nephrosonephritis. KHF is endemic to areas of Russia, China and Korea. Hemorrhagic Fever with Renal Syndrome (HFRS) is a debilitating and occasionally fatal disease of humans.

RNA viruses are known to cause malignant neoplasms in a wide range of species (Robinson, H.L., Rev. Infect. Dis. Volume 4, pages 1015-1025 (1982)). One such RNA virus named human T-cell Leukemia Virus (HTLV) has been associated with malignant proliferation of human T-lymphocytes. Identification of HTLV-like particles in patients with Acquired Immune Deficiency Syndrome (AIDS) also implicates this virus in the etiology of AIDS, which is characterized by immunodeficiency and the development of Kaposi's sarcoma. Furthermore, these patients are susceptible to various infections. (Essex, M., et al., Science, Volume 220, pages 859-871 (1983)).

Retrovirus-like particles containing RNA-directed DNA polymerase (reverse transcriptase) have also been isolated from human prostatic cells (Arya, S.K., et al., Oncology, Volume 37, pages 129-135 (1980)). Moreover, RNA-dependent polymerase activity has been found in human granulocytic sarcoma, human primary melanoma and human osteosarcoma tissue. (Chandra, P., Proc. Fed. Eur. Biochem. Soc., Volume 61, pages 215-218 (1980)). These studies suggest RNA oncogenic viruses as potential causative agents in certain types of human cancers.

In Higgins et al. (J. Antimicrob. Chemother. (1984), Suppl. A, 27-41), significant inhibitory effects of Ribavirin against Rift Valley Fever virus, the Bunyavirus Punta Toro, Hantaan virus and Arenaviruses such as Pichinde, Junin, Machupo and Lassa Fever were shown by using laboratory animal models. However, Ribavirin and certain analogues had no detectable *in vivo* activity against arboviral encephalitic infections caused by Venezuelan or Western Equine Encephalitis or Semliki Forest Viruses.

Furthermore, in Nicholson (The Lancet (1984) 9, 562), the broad spectrum of antiviral activity of Ribavirin was discussed. Ribavirin had a modest effect on cellular DNA synthesis, but was cytotoxic to resting cell lines only at concentrations of 200-1000 µg/ml.

In Heel & Chang (Drugs (1981) 22: 111-128), it was reported again about Ribavirin's usually wide spectrum of antiviral activity. A large number of RNA and DNA viruses were sensitive in in vitro tests, especially herpes virus, poxvirus, influenza, parainfluenza, reovirus, togavirus, and RNA tumor virus. The in vivo antiviral spectrum of activity was much narrower, with activity against herpes virus, influenza, parainfluenza, measels and adenoviruses. However, controlled clinical trials had not been uniformly successful in treating influenza, hepatitis, herpes simplex and herpes zoster.

In Pannier et al. (Antiviral Res. (1982) 2(6): 331-337), a comparison of ribavirin with another antiviral drug, pyrazofurin is provided. Both compounds markedly inhibited the in vitro replication of a number of RNA viruses including Rift Valley fever (RVF), Venezuelan equine encephalomyelites (VEE), Sandfly, Pichinde, Lassa and LCM virus. Even though neither pyrazofurin nor ribavirin have shown in vivo tests that these compounds protect mice and guinea pigs against a lethal challenge with VEE and Pichinde virus, respectively, none of the presented results provide any evidence which would indicate that ribavirin can be used for treating arboviral diseases in high doses.

To summarize, no reliable data had been onbtainable with respect to the in-vivo use and efficacy, in particular a high dosage in-vivo treatment of diseases caused by Arboviruses.

The feature that unites RNA tumor viruses (retroviruses) and distinguishes them from all other animal viruses is the transcription of their single-stranded RNA into double stranded DNA and the presence of reverse transcriptase, an RNA-dependent DNA polymerase that is found in all RNA oncogenic viruses. Mutant RNA viruses lacking reverse transcriptase lose their ability to initiate infection and cell transformation. The presence of reverse transcriptase in all oncornaviruses strongly suggests its role in the neoplastic transformation of such viruses.

Therefore, the present invention involves the use of the compound Ribavirin 1-β-D-ribofuranosyl-1,2,4-triazole-3-carboxamide for the manufacture of a pharmaceutical composition for the medical treatment of viral diseases in humans, caused by Arboviruses with the proviso that Bunyaviruses are excluded, wherein the compound 1-β-D-ribofuranosyl-1,2,4-triazole-3-carboxamide is contained in said pharmaceutical composition in a total dose from 8.5 to 50 milligrammes per kilogramme of body weight per day.

It is preferred that the pharmaceutical composition comprising the compound 1-β-D-ribofuranosyl-1,2,4-triazole-3-carboxamide is used for the treatment of a human, wherein said human has a compromised immune system in addition to said viral disease.

Another preferred embodiment of the invention refers to the hereinbefore described uses of the compound 1-β-D-ribofuranosyl-1,2,4-triazole-3-carboxamide, wherein the compound is present in the pharmaceutical composition in an amount of 0.01% to 50% by weight based on the total weight of the composition.

The compound can be administered to the human patient by injection, orally, topically, opthalmically, or via the respiratory tract in aerosols or drops. For systemic use, the compound would be given in an amount such that the total daily dose of the compound is from about 50 milligrammes to about 2500 milligrammes. The normal range for use according to the invention is from 8.5 to 50 milligrammes per kilogram of body weight per day. Depending on the mode of administration, the compound can be formulated with appropriate diluents to form solutions, suspensions, tablets, capsules, or syrups. For topical and ophthalmic use, the compound can be formulated with the appropriate diluents and carriers to form ointments, creams, foams, and solutions having from 0.01% to 15% by weight, preferably from 1% to 10% by weight of the compound. In any event, the actual amount should be sufficient to provide a chemotherapeutically effective amount of the compound to the patient, all of which will be readily within the ability of those skilled in the art to determine given the disclosure herein.

## Claims

1. The use of the compound 1-β-D-ribofuranosyl-1,2,4-triazole-3-carboxamide for the manufacture of a pharmaceutical composition for the medical treatment of viral diseases in humans, caused by Arboviruses, with the proviso that Bunyaviruses are excluded, wherein the compound 1-β-D-ribofuranosyl-1,2,4-triazole-3-carboxamide is contained in said pharmaceutical composition in a total dose from 8.5 to 50 milligramms per kilogramme of body weight per day.

2. The use of claim 1, wherein said human have a compromised immune system in addition to said viral disease.

3. The use according to any of claims 1 to 2, wherein the compound is present in the pharmaceutical composition in an amount of 0.01% to 50% by weight based on the total weight of the composition.

4. The use according to any of the foregoing claims, wherein the composition is in a form suitable for oral administration, topical administration, administration by injection or administration via the respiratory tract.

## Patentansprüche

1. Verwendung der Verbindung 1-ß-D-Ribofuranosyl-1,2,4-triazol-3-carboxamid zur Herstellung einer pharmazeutischen Zusammensetzung für die medizinische Behandlung viraler, von Arboviren verursachter Krankheiten in Menschen, mit der Maßgabe, daß Bunyaviren ausgeschlossen sind, wobei die Verbindung 1-β-D-Ribofuranosyl-1,2,4-triazol-3-carboxamid in der pharmazeutischen Zusammensetzung in einer Gesamtdosis von 8,5 bis 50 Milligram pro Kilogram Körpergewichtes pro Tag.

2. Verwendung gemäß Anspruch 1, wobei neben der viralen Krankheit die Menschen ein geschwächtes Immunsystem haben.

3. Verwendung gemäß einem der Ansprüche 1 bis 2, wobei die Verbindung in der pharmazeutischen Zusammensetzung in einer Menge von 0,01 Gew.% bis 50 Gew.% bezogen auf das Gesamtgewicht der Zusammensetzung enthalten ist.

4. Verwendung gemäß einem der vorangegangenen Ansprüche, wobei die Zusammensetzung in einer Form ist, die zur oralen Verabreichung, äußerlichen Verabreichung, Verabreichung durch Injektion oder Verabreichung über den Atemweg geeignet ist.

## Revendications

1. Utilisation du composé 1-β-D-ribofuranosyl-1,2,4-triazole-3-carboxamide pour la fabrication d'une composition pharmaceutique pour le traitement médical de maladies virales chez l'homme, causées par les Arbovirus, à l'exclusion des Bunyavirus, dans laquelle le composé 1-β-D-ribofuranosyl-1,2,4-triazole-3-carboxamide est contenu dans ladite composition pharmaceutique dans une dose totale de 8,5 à 50 milligrammes par kilogramme de poids corporel et par jour.

2. Utilisation selon la revendication 1, dans laquelle ledit humain a un système immunitaire affaibli en plus de ladite maladie virale.

3. Utilisation selon l'une des revendications 1 à 2, dans laquelle la composition est présente dans la composition pharmaceutique dans une quantité de 0,01 % à 50 % en poids par rapport au poids total de la composition.

4. Utilisation selon l'une des revendications précédentes, dans laquelle la composition est sous une forme appropriée pour l'administration orale, l'administration topique, l'administration par injection ou l'administration par les voies respiratoires.
